# EUROPEAN PATENT APPLICATION

(11) **EP 0 761 686 A2**
(43) Date of publication of application: **12.03.1997**
(21) Application number: 96113399.8
(22) Date of filing: 21.08.1996
(51) Int. Cl.: C07K 14/745, A61K 38/36

(54) **Anticoagulant factor Va derivatives**

(30) Priority: 29.08.1995 US 520725
(71) Applicant: IMMUNO Aktiengesellschaft, A-1221 Wien (AT)
(72) Inventor: Rosing, Jan, Dr., 6301 VK Valkenburg (NL); Tans, Guido, Dr., 6215 XK Maastricht (NL); Schwarz, Hans-Peter, Doz., 1180 Wien (AT); Varadi, Katalin, Dr., 1230 Wien (AT); Redl, Gerda, Dr., 2301 Rutzendorf (AT)
(74) Representative: Kolb, Helga, Dr. Dipl.-Chem.

(57) **Abstract**

Anticoagulant factor Va derivatives are described which can be used in treating patients for a hypercoagulant condition, thrombosis or thromboembolic disease.

There is also disclosed a method for preparing novel anticoagulant factor Va derivatives.

## Description

The present invention relates to isolated anticoagulant factor Va derivatives, as well as to methods for preparing the factor Va derivatives and for using them to treat patients for a hypercoagulant condition, thrombosis or thromboembolic disease.

### Background of the Invention

Human blood coagulation factor Va is a heterodimeric glycoprotein that includes a heavy chain (Mr=105,000) and a light chain (Mr=72,000-74,000). Suzuki *et al*., *J. Biol. Chem*. 257: 6556-6564 (1982); Rosing *et al.*, *loc. cit*. 268: 21130-21136 (1993). The heavy and light chains are non-covalently associated together by divalent metal ions, such as Ca²⁺.

The amino acid sequence of human factor V is described in Jenny *et al., Proc. Nat'l Acad. Sci. USA* 84: 4846-50 (1987), and the various regions of factor V are discussed in Kalafatis *et al., J. Biol. Chem.* 269: 31869-80. The heavy chain region (amino acids 1-709) is composed of two A domains (A1 and A2) associated with a connecting region (amino acids 304-316). The light chain region of the factor (amino acids 1546-2196) includes one A and two C domains (A3, C1, C2). The heavy and light chain regions are connected in factor V through the connecting B region, residing between amino acids 709 and 1546, which is removed during activation to form activated factor V (factor Va). Factor Va is a cofactor of activated factor X (factor Xa), and accelerates the factor Xa-catalysed formation of thrombin from prothrombin over 1000-fold. Ichinose *et al., Hemostas. Thromb.* at 26 (Coleman *et al.* eds. 1994).

Proteolytic inactivation of factor Va by activated protein C (APC) is one of the key reactions in the regulation of thrombin formation. APC-catalyzed cleavage of factor Va is stimulated by the presence of negatively-charged membrane surfaces and by protein S. Walker *et al.*, *Biochim. Biophys. Acta* 571: 333-42 (1979); Suzuki *et al.*, *J. Biol. Che*m 258: 1914-20 (1983); Bakker *et al.*, *Eur. J. Biochem.* 208: 171-78 (1992); Walker, *J. Biol. Chem.* 255: 5521-24 (1980); Solymoss *et al.*, *loc. cit.* 263: 14884-90 (1988). The loss of cofactor activity by factor Va is associated with peptide bond cleavages in its heavy chain at Arg³⁰⁶, Arg⁵⁰⁶, and Arg⁶⁷⁹. Kalafatis *et al.*, *J. Biol. Chem.* 269: 31869-80 (1994); *loc. cit.* 270: 4054-57 (1995). The physiologic importance of the down-regulation of factor Va activity by APC is underscored by the observation of recurrent thromboembolic events in individuals that are deficient in either protein C or protein S. Griffin *et al.*, *J. Clin. Invest.* 68: 1370-73 (1981), *Schwarz et al.*, *Blood* 64: 1297-1300 (1984), Comp *et al.*, *J. Clin. Invest.* 74: 2082-88 (1984).

There have been reports that cleavage at Arg⁵⁰⁶ alone in normal factor Va has no effect on cofactor activity. But this cleavage is necessary for efficient exposure of cleavage sites at Arg³⁰⁶ and Arg⁶⁷⁹, which are associated with cofactor inactivation. Arg³⁰⁶ is in the A1 region, Arg⁵⁰⁶ is in the A2 region, and Arg⁶⁷⁹ is in a small part of the B region that remains in the heavy chain of factor Va after activation. Kalafatis *et al., supra* (1994).

In the absence of a membrane surface, APC cleaves factor Va heavy chain to generate a fragment of 75 kD, as well as 28/26 kD and 22/20 kD doublets. Further fragmentation of the heavy chain is possible only in the presence of phospholipids from membrane surfaces, however. In the presence of such surfaces, APC quickly inactivates factor Va.

Several laboratories have associated the occurrence of familial thrombophilia in a large group of patients with a poor anticoagulant response to APC (APC resistance). See, for example, Dahlbäck *et al.*, *Proc. Nat'l Acad. Sci. USA* 90: 1004-08 (1993). APC resistance is at least 10 times more common than all other known genetic thrombosis risk factors together and has an allelic frequency of about 2% in the Dutch population. Koster *et al.*, *Lancet* 342: 1503-06 (1993). A molecular defect in APC-resistant patients was recently demonstrated to be linked to a single point mutation in the factor V gene that causes an amino acid substitution (Arg⁵⁰⁶→Gln), which is a site that is cleaved during APC-catalyzed inactivation of native factor Va. Bertina *et al.*, *Nature* 369: 64-67 (1994); Greengard *et al.*, *Lancet* 343: 1361-62 (1994); Voorberg *et al., Lancet* 343: 1535-36 (1994); Kalafatis *et al.*, *J. Biol. Chem.* 269: 31869-80 (1994).

### Summary of the Invention

It is an object of the present invention to provide an isolated factor Va derivative that has anticoagulant properties.

It is another object of the present invention to provide an isolated factor Va derivative that has a reduced affinity for factor Xa proteins, including native factor Xa, factor Xa-like polypeptides, and factor Xa with reduced serine esterase activity, but still can interact with prothrombin to interfere with the formation of a functional, prothrombin-activating complex.

It is a further object of the invention to provide factor Va derivatives in a purified state.

It is still another object to provide a treatment, using a factor Va derivative, for a patient at risk for or suffering from a hypercoagulant condition, thrombosis or a thromboembolic disease.

In accomplishing these and other objects, there has been provided, in accordance with one aspect of the present invention, an isolated anticoagulant factor Va derivative that has reduced affinity for factor Xa proteins and that comprises a truncated heavy chain of factor Va linked to a light chain of factor Va, wherein the truncated heavy chain comprises an A1 domain and a portion of an A2 domain. Preferably, the factor Va derivative has a heavy chain that is a 75 kD fragment obtainable by cleavage of the heavy chain at Arg⁵⁰⁶. The factor Va derivative also can be associated with a 26/28 kD carboxy-terminal fragment obtainable by cleavage of the heavy chain at Arg⁵⁰⁶. The cleavage can be effected by a serine protease, such as activated protein C.

In accordance with another aspect of the present invention, a pharmaceutical preparation is provided that comprises a factor Va derivative. Preferably, the preparation is treated to remove or inactivate pathogens, such as bacteria and viruses.

In accordance with still another aspect of the present invention, there are provided methods of treating patients for hypercoagulant conditions, thromboses or thromboembolic diseases with factor Va derivatives.

These and other aspects of the invention will become apparent to the skilled artisan in view of the teachings contained herein.

### Detailed Description of Preferred Embodiments

An anticoagulant factor Va derivative according to the present invention may be obtained by cleavage of the heavy chain at Arg⁵⁰⁶ by using the serine protease APC. Other serine proteases capable of cleaving the heavy chain at this site or nearby sites also can be used. Similar fragments that contain the A1 domain and a partial A2 domain can be obtained with other approaches, such as expression by recombinant DNA technology.

Preferably, the two chains of the factor Va derivative are linked together via a calcium ion bridge, or other divalent cation bridges containing magnesium, manganese or strontium. Factor Va derivatives where the heavy chain and the light chain are linked together by covalent bonds, such as peptide bonds, also are within the present invention.

The inventive factor Va derivatives have an anticoagulant effect because they are unable to assemble into a functional prothrombinase complex. Accordingly, the invention not only relates to fragments of native factor Va, but also to factor Va mutants and chemically synthesized derivatives of factor Va that have these properties.

For example, changes in the amino acid sequence of the factor Va derivatives are contemplated in the present invention. The factor Va derivative can be altered by changing the DNA encoding the protein. Preferably, only conservative amino acid alterations, using amino acids that have the same or similar properties, are undertaken. Illustrative amino acid substitutions include the changes of: alanine to serine; arginine to lysine; asparagine to glutamine or histidine; aspartate to glutamate; cysteine to serine; glutamine to asparagine; glutamate to aspartate; glycine to proline; histidine to asparagine or glutamine; isoleucine to leucine or valine; leucine to valine or isoleucine; lysine to arginine, glutamine, or glutamate; methionine to leucine or isoleucine; phenylalanine to tyrosine, leucine or methionine; serine to threonine; threonine to serine; tryptophan to tyrosine; tyrosine to tryptophan or phenylalanine; valine to isoleucine or leucine.

Additionally, other variants and fragments of the factor Va derivative can be used in the present invention. Variants include analogs, homologs, derivatives, muteins and mimetics of the factor Va derivative protein that retain the ability to cause the beneficial results described herein. Fragments of the factor Va derivative refer to portions of the amino acid sequence of the factor Va derivative that also retain this ability. The variants and fragments can be generated directly from the factor Va derivative itself by chemical modification, by proteolytic enzyme digestion, or by combinations thereof. Additionally, genetic engineering techniques, as well as methods of synthesizing polypeptides directly from amino acid residues, can be employed.

Non-peptide compounds that mimic the binding and function of the factor Va derivative ("mimetics") can be produced by the approach outlined in Saragovi *et al.*, *Science* 253: 792-95 (1991). Mimetics are molecules which mimic elements of protein secondary structure. See, for example, Johnson *et al.*,"Peptide Turn Mimetics," in BIOTECHNOLOGY AND PHARMACY, Pezzuto *et al.*, Eds., (Chapman and Hall, New York, 1993). The underlying rationale behind the use of peptide mimetics is that the peptide backbone of proteins exists chiefly to orient amino acid side chains in such a way as to facilitate molecular interactions. For the purposes of the present invention, appropriate mimetics can be considered to be the equivalent of the factor Va derivative itself.

Variants and fragments also can be created by recombinant techniques employing genomic or cDNA cloning methods. Site-specific and region-directed mutagenesis techniques can be employed. See CURRENT PROTOCOLS IN MOLECULAR BIOLOGY vol. 1, ch. 8 (Ausubel *et al.* eds., J. Wiley & Sons 1989 & Supp. 1990-93); PROTEIN ENGINEERING (Oxender & Fox eds., A. Liss, Inc. 1987). In addition, linker-scanning and PCR-mediated techniques can be employed for mutagenesis. See PCR TECHNOLOGY (Erlich ed., Stockton Press 1989); CURRENT PROTOCOLS IN MOLECULAR BIOLOGY, vols. 1 & 2, *supra*. Protein sequencing, structure and modeling approaches for use with any of the above techniques are disclosed in PROTEIN ENGINEERING, *loc. cit.*, and CURRENT PROTOCOLS IN MOLECULAR BIOLOGY, vols. 1 & 2, *supra*.

If the compounds described above are employed, the skilled artisan can routinely insure that such compounds are amenable for use with the present invention in view of the screening techniques described herein. The screening techniques include tests for a partial cofactor activity of factor Va. If the compounds described above bind to a part of the prothrombinase complex, but are unable to assemble to the functional prothrombinase complex, they are suitable according to the invention.

Surprisingly, the factor Va derivatives according to the present invention have sufficient stability to permit chromatographic purification. Following specific cleavage of factor Va heavy chain at Arg⁵⁰⁶, the 75 kD fragment is obtained, which is still associated with the light chain of factor Va. The 75 kD fragment can be associated with the 26/28 kD carboxy-terminal fragment of the heavy chain, which also can be associated with the light chain. These fragments are formed after cleavage of the heavy chain by APC at Arg⁵⁰⁶, and can remain non-covalently associated in the factor Va derivative. Gel electrophoretic analysis shows that the column fractions in which the factor Va derivative elute contain both the 75 kD and 26/28 kD fragments of the heavy chain.

The factor Va derivative is partially inactivated, and thus retains a partial cofactor activity. The complete cofactor activity of factor Va is dependent on its interaction with procoagulant membranes (phospholipids), factor Xa, prothrombin and on its ability to increase the catalytic activity of factor Xa. The factor Va derivative according to the present invention unexpectedly has an unimpaired affinity to bind to prothrombin. Its affinity for factor Xa proteins is significantly reduced, however, which means that the factor Va derivative fails to assemble with factor Xa proteins into a membrane bound prothrombin activating complex (prothrombinase complex). Accordingly, the factor Va derivatives according to the present invention retain some, but not all, activities of the native factor Va, and can be considered to be partially inactivated. The vast reduction in the ability of the factor Va derivative to interact with factor Xa proteins forms the basis of the anticoagulant effect of this inventive compound. The factor Va derivative according to the invention is therefore characterized by a reduced affinity to factor Xa proteins, such as native factor Xa, factor Xa-like polypeptides, for example, fragments, analogs, homologs, muteins, mimetics, etc., and factor Xa with reduced serine esterase activity. Factor Xa molecules having a reduced serine esterase activity are generally known. See U.S. patent No. 5,120,537.

The cofactor activity of the factor Va derivative cleaved at Arg⁵⁰⁶ strongly depends on the factor Xa concentration in the prothrombin activation mixture in which factor Va is assayed. Cofactor activity of the factor Va derivative in prothrombin activation is only observed at high factor Xa concentrations. Compared with native factor Va, full expression of cofactor activity of factor Va that is cleaved at Arg⁵⁰⁶ requires a 45-fold higher factor Xa concentration. This observation indicates that cleavage at Arg⁵⁰⁶ in the heavy chain of factor Va effects its interaction with factor Xa and results in a considerable loss of affinity for factor Xa proteins.

The factor Va derivative according to the invention is preferably isolated. The term "isolated" in the context of proteins denotes separation of the protein from its original source or surroundings.

Preferably, the factor Va derivative is brought to a more purified state. Thus, it is especially preferred that the factor Va derivative be substantially free of other proteins, such as native factor Va or activation by-products, which otherwise would be present when the derivative is produced from factor V. Typically, the presence of contaminating proteins is eliminated or greatly reduced.

A factor Va derivative within the present invention can be purified by a variety of techniques, including gel electrophoresis (SDS-PAGE), isoelectric focusing, ion exchange chromatography, gel exclusion chromatography, affinity chromatography, immunoprecipitation, and combinations thereof.

The purity of the factor Va derivative in a preparation according to the invention can be determined by SDS-PAGE, and is expressed in percentage of protein (w/w). It is preferred that a preparation of the factor Va derivative according to the invention be at least 85% pure, and preferably at least 95% pure in terms of total protein. Preferably, the purified factor Va derivative preparation contains less than 5% native factor Va. The purified factor Va derivative may be then incorporated in a pharmaceutical composition, optionally including other components, such as blood factors affecting coagulation or fibrinolysis.

When the factor Va derivative is purified by chromatographic means, native factor Va, and proteins and fragments of factor V or Va that result from activation and/or inactivation of the protein can be removed efficiently. Additionally, the activation fragment, which is the by-product peptide fragment that is lost during the activation procedure, for example, the B-domain, amino acids 664-709, can be removed. The purified preparation of factor Va therefore contains less than 5% native factor Va and no detectable activation fragment.

The chromatographic purification is preferably carried out using gel chromatography and/or ion exchange chromatography. If ion exchange is employed, it is preferably carried out on a column filled with monobeads, such as Mono Q or Mono S, or other appropriate materials. Mono Q (Pharmacia) is a strong anion exchanger. Mono S is a strong cation exchanger with charged sulfonic groups. The chromatographic materials usually are based upon a carbohydrate or a synthetic polymer carrier having ligands, which specifically bind to the factor Va derivative. The ligands also can contain ion exchange groups, such as DEAE, TMAE, TEAE, QAE, Q-groups or sulfonic groups, but also aminoalkyl groups. Affinity ligands usually are selected from specific antibodies against the heavy or the light chain of factor Va.

The factor Va derivative according to the invention can be formulated to obtain a pharmaceutical preparation which contains a pharmaceutically acceptable carrier. Pharmaceutically acceptable carriers include aqueous solutions, non-toxic excipients, including salts, preservatives, buffers and the like, as described in REMINGTON'S PHARMACEUTICAL SCIENCES, 15th Ed. Easton: Mack Publishing Co. pp 1405-1412 and 1461-87 (1975) and THE NATIONAL FORMULARY XIV., 14th Ed. Washington: American Pharmaceutical Association (1975), the contents of which are hereby incorporated by reference. Examples of non-aqueous solvents are propylene glycol, polyethylene glycol, vegetable oil and injectable organic esters, such as ethyloleate. Aqueous carriers include water, alcoholic/aqueous solutions, saline solutions, parenteral vehicles such as sodium chloride, Ringer's dextrose, etc. Intravenous vehicles include fluid and nutrient replenishers. Preservatives include antimicrobials, anti-oxidants, chelating agents and inert gases. The pH and exact concentration of the various components of the binding composition are adjusted according to routine skills in the art. *See* GOODMAN AND GILMAN'S THE PHARMACOLOGICAL BASIS FOR THERAPEUTICS (7th ed.).

The pharmaceutical preparation also can include stabilizing proteins, amino acids or buffers. It is preferred to stabilize the pharmaceutical preparation with albumin. Preferably, the pharmaceutical preparation according to the invention contains the factor Va derivative as the sole active ingredient for anticoagulation. The factor Va derivative can be used with other anticoagulants, however.

Preferably, the factor Va derivative is contained as a purified protein in the pharmaceutical preparation. Other biologically-active derivatives of factor Va may also be included, and stabilized as explained above.

A factor Va derivative according to the present invention is preferably of human origin. Other sources, such as pigs and cattle, also are appropriate.

Preferably, a factor Va derivative according to the invention, or the source material used to produce the factor Va derivative, is treated to inactivate any pathogens, such as bacteria and viruses, that may be present. Heat treatment in a liquid or solid state, or other reliable virus inactivation measures, such as treatment with detergents or chaotropic agents, should be undertaken. See, for example, EP 0 159 311, EP 0 519 901, WO 94/13329, DE 4 434 538, EP 0 131 740, WO 90/15613.

According to the invention a patient at risk for or suffering from a hypercoagulant condition, thrombosis or thromboembolic disease is treated by administering an effective amount of a factor Va derivative that has the above-described character. The term "treating" in its various grammatical forms in relation to the present invention refers to preventing, curing, reversing, attenuating, alleviating, minimizing, suppressing or halting the deleterious effects of a disease state or progression. The factor Va derivative according to the invention is formulated to a preparation suitable for parenteral or mucosal administration. It is preferred to administer the preparation via i.v., i.m., i.d. or subcutaneous routes as a bolus injection or continuous infusion.

The factor Va derivative acts as a competitor of factor V/factor Va, which is normally present *in vivo* and thus interferes with the formation of the prothrombinase complex. As a consequence, the activation of prothrombin to thrombin is greatly reduced, which in turn minimizes blood clot formation and aggregation of blood platelets.

The normal amount of factor V is about 10µg/blood. The effective amount of factor Va derivative to act as an anticoagulant usually is in the range of 10ng-100µg factor Va derivative/ml of blood, preferably more than 0.1µg. The level of factor Va in blood can be determined by common means, for instance, by a suitable immunoassay or ELISA technique.

The effective amount of the factor Va derivative, for example, corresponds to the amount that results in a normalization of the APC response in the patient suffering from a reduced APC response. The APC response can be determined using a test for the sensitivity to APC according to EP 0 656 424 or a test for APC resistance according to WO 93/10261. In patients who have normal coagulation responses, APC would inactivate factor Va and prevent formation of a functional prothrombinase complex. With patients who have APC resistance or lack of sensitivity, the functional factor Va is not inactivated properly and, therefore, a factor Va derivative is needed to antagonize or interfere with the functional factor Va in order to prevent formation of a functional prothrombinase complex.

A dose for the factor Va derivative to correct a hypercoagulant condition, thrombosis or thromboembolic disease is calculated on the basis of suitable *in vitro* or *in vivo* assays. For instance, a plasma coagulation parameter, like aPTT, is determined presence and absence of added APC for diagnosing APC resistance. The action of the factor Va derivative as an anticoagulant can be then quantified by determining the amount of factor Va derivative that is required to correct the value for the plasma coagulation parameter of a patient's plasma in the presence of APC to the value determined for normal plasma. Besides APC resistance or lack of sensitivity, other indications for the anticoagulant factor Va derivative include lupus, the presence of anti-phospholipid antibodies or any other cause for the enhanced prothrombinase action.

The invention is further explained by the following, non-limiting examples.

### I. Preparation of a factor Va derivative after cleavage at Arg⁵⁰⁶ by APC

The factor Va derivative was obtained from a mixture that already contained purified factor Va as a starting product.

The first steps of the preparation of human factor V were essentially as described by Dahlbäck, *J. Clin. Invest.* 66: 583-91 (1980), with minor modifications according to Tans *et al.*, *Blood* 77: 2641-48 (1991). The factor V obtained was applied to a Resource S column and eluted as a purified product. For the activation of factor V, a fraction containing the purified factor V (33 µg/ml) was incubated with 5 nM thrombin in a buffer solution (25 mM Hepes, 5 mM CaCl₂, 175mM NaCl, pH 7.5) for 20 minutes. After activation, 15 nM PPACK was added to inhibit thrombin. Factor Va was further subjected to fast protein liquid chromatography (FPLC) on a Mono S column (HR 5/5) at room temperature. Rosing *et al., supra*. The factor Va bound to the Mono S column was eluted with 15 ml of a linear gradient (50 mM-1000mM NH₄Cl) in 25 mM Hepes (pH 7.5), 5 mM CaCl₂. Factor Va activity eluted from the column in two well-separated protein peaks containing factor Va with light chains of Mr=74,000 and Mr=71,000, respectively. Rosing *et al., supra*. The two factor Va-containing peaks were pooled and brought into 25 mM Hepes (pH 7.5), 175 mM NaCl, 5 mM CaCl₂ by a buffer exchange on a PD-10 column.

The factor Va derivative was obtained by inactivation of the factor Va using APC in the absence of phospholipids. Purified human factor Va (40 nM) was incubated with 15 nM APC in 25 mM Hepes (pH 7.5), 175 mM NaCl, 3 mM CaCl₂ and 5 mg/ml BSA at 37°C.

After completion of the first phase of inactivation by Arg⁵⁰⁶ cleavage, which takes about 45 minutes, the reaction mixture was diluted 10-fold with 25 mM Hepes (pH 7.5), 50 mM NH₄Cl, 5 mM CaCl₂ and 1 mg/ml BSA.

### II. Purification of the factor Va derivative

The dilute inactivation mixture obtained according to example I was applied to a Mono S column (HR 5/5). The column was washed with 10 ml of a butter containing 25 mM Hepes (pH 7.5), 50 mM NH₄Cl and 5 mM CaCl₂. Bound protein was eluted with 20 ml of a linear NH₄Cl gradient (50-1000 mM) in 25 mM Hepes (pH 7.5) and 5 mM CaCl₂. The factor Va derivative eluted between 700-800 mM NH₄Cl. Purity of the factor Va derivative was analyzed by SDS-PAGE on 8% acrylamide slabgels, electrophoretic transfer of proteins from the gel to Immobilon-P membranes and identification of heavy chain and heavy chain fragments with a polyclonal rabbit-antibody directed against the heavy chain of factor Va and goat-anti-rabbit IgG conjugated with alkaline phosphatase.

Intact factor Va heavy chain was essentially eliminated. Visual inspection of the gel indicated that the factor Va derivative was obtained with more than 95% purity.

### III. Cofactor activity of the factor Va derivative

Cofactor activities of native factor Va and the factor Va derivative obtained according to Example II were quantified by determining the rate of factor Xa-catalyzed prothrombin activation in reaction mixtures that contained a limiting amount of factor Va or factor Va derivative, purified human factor Xa, 50 µM phospholipid vesicles (DOPS/DOPC, 10/90, mol/mol) and 0.5 µM human prothrombin. Rates of prothrombin activation were determined with the chromogenic substrate S2238. When the assay was performed at 0.1 nM factor Xa, the cofactor activity of the factor Va derivative was at least 40-fold lower than that of native factor Va.

From these data it was determined that APC-catalyzed cleavage at Arg⁵⁰⁶ of the heavy chain of factor Va resulted in the formation of a factor Va derivative that has lost part of its cofactor activity because of a reduction of its affinity for factor Xa.

The interaction of the membrane-bound factor Xa-Va complex with prothrombin was not affected by cleavage at Arg⁵⁰⁶ since the Kₘ values for prothrombin were virtually the same for prothrombinase complexes with normal and cleaved factor Va (data not shown).

It is to be understood that the description, specific examples and data, while indicating preferred embodiments, are given by way of illustration and exemplification and are not intended to limit the present invention. Various changes and modifications within the present invention will become apparent to the skilled artisan from the discussion and disclosure contained herein.

## Claims

1. An isolated anticoagulant factor Va derivative that has reduced affinity for factor Xa proteins and that comprises a truncated heavy chain of factor Va linked to a light chain of factor Va, wherein said heavy chain comprises an A1 domain and a portion of an A2 domain.

2. An isolated anticoagulant factor Va derivative according to claim 1, obtainable by partial inactivation of factor Va in the presence of a serine protease.

3. An isolated anticoagulant factor Va derivative according to claim 2, said partial inactivation being effected by activated protein C.

4. An isolated anticoagulant factor Va derivative having a heavy chain and light chain from factor Va, wherein said heavy chain is a 75 kD fragment obtainable by cleavage of the heavy chain at Arg⁵⁰⁶.

5. An isolated anticoagulant factor Va derivative according to claim 4, wherein said derivative is associated with a 26/28 kD carboxy-terminal fragment obtainable by cleavage of the heavy chain at Arg⁵⁰⁶.

6. An isolated anticoagulant factor Va derivative according to one or more of the preceding claims 1 to 5, said anticoagulant factor Va derivative being capable of inhibiting the formation of a functional prothrombinase complex by interacting with prothrombin and phospholipids.

7. An isolated anticoagulant factor Va derivative according to one or more of preceding claims 1 to 6, said anticoagulant factor Va derivative having a reduced affinity for factor Xa proteins.

8. A pharmaceutical preparation comprising an effective amount of factor Va derivative according to one or more of claims 1 to 7 and a pharmaceutically acceptable carrier.

9. A pharmaceutical preparation according to claim 8, wherein said factor Va derivative is at least 85 % pure.

10. A pharmaceutical preparation according to claim 8 or 9, wherein said preparation has been treated to inactivate contaminating pathogens.

11. A method for preparing a factor Va derivative according to one or more of claims 1 to 7, comprising the steps of:
- incubating factor Va with a serine protease to form said factor Va derivative; and optionally
- purifying said factor Va derivative by chromatography.

12. A method according to claim 11, wherein said serine protease is activated protein C, and said incubating is carried out in the absence of a phospholipid.

13. A method according to claim 11 or 12, wherein said factor Va derivative is purified by gel chromatography.

14. The use of an isolated anticoagulant factor Va derivative according to one or more of claims 1 to 7 for the preparation of a pharmaceutical useful for treating hypercoagulant condition, thrombosis or a thromboembolic disease.

15. The use according to claim 14, wherein said disease is caused by a poor anticoagulant response to activated protein C.

16. The use according to claim 14, wherein said disease is lupus or is caused by anti-phospholipid antibodies.

17. The use according to one or more of claims 14 to 16, wherein said effective amount is 10ng to 100mg factor Va derivative/ml of blood.

18. The use of an isolated anticoagulant factor Va derivative according to one or more of claims 1 to 7 for the preparation of a pharmaceutical useful for inhibiting coagulation in the case of abnormal blood condition selected from the group consisting a hypercoagulant condition, a thrombosis or a thromboembolic disease.
